# EUROPEAN PATENT APPLICATION

(11) **EP 4 206 991 A1**
(43) Date of publication of application: **05.07.2023**
(21) Application number: 22216168.9
(22) Date of filing: 22.12.2022
(51) Int. Cl.: G06N 3/04, G06N 3/08, G16H 50/00, A01K 27/00, A01K 29/00, A61B 5/00, A61B 5/024, A61B 5/0507, A61B 5/08, A61B 5/11, A61B 5/113

(54) **AN INTELLIGENT DOG COLLAR FOR MONITORING PHYSIOLOGICAL PARAMETERS OF A DOG**

(30) Priority: 30.12.2021 EP 21218417
(71) Applicant: Invoxia, 92130 Issy les Moulineaux (FR)
(72) Inventor: HUMBERT, Eric, 92100 BOULOGNE BILLANCOURT (FR); CAUDRON, Amélie, 75019 PARIS (FR)
(74) Representative: Plasseraud IP

(57) **Abstract**

An intelligent dog collar for monitoring physiological parameters of a dog, comprising:
- a movement sensor unit (12) comprising an accelerometer and/or a gyrometer, wherein the movement sensor unit (12) is configured to detect raw movement signals (13) of the dog collar (1),
- a storage module (15) storing a trained neural network (16), the neural network (16) being configured to determine a physiologic information into raw movement signals (13) detected by the movement sensor unit (12),
- a processing unit (5) connected to the movement sensor unit and configured to operate the trained neural network,
- a memory (6) configured to store the identified physiologic information,
- an interface (17) for transmitting to a communication device (18) the identified physiologic information.

## Description

### Technical Field

This disclosure pertains to the field of intelligent dog collars for monitoring physiological parameters of a dog.

### Background Art

Owning a dog usually represents a great emotional investment. However, as good as the link between the dog owner and the dog may be, most of the time, the dog owner cannot guess that its dog has a heart problem or breathing problem, because dogs do not speak.

However, 10% of the dogs do have heart or breathing problems. Sometimes, the owner or the veterinarian take some measures, either manual or with dedicated medical devices into the veterinary clinic, but those are merely occasional.

In orderto continuously monitor the heart or breathing rate, there is a need for a non-invasive wearing device that can be worn by the dog without any drawback health hazard or discomfort.

The technologies usually used in order to monitor the health of human beings are not easily usable for dogs, because of hairs which are obstacles for most signals and induce noises/artifacts in the signal.

EP3089654 teaches a dog collar for monitoring the heart or breathing rate of a dog, but the frequencies used in the ultra-wideband radar may be non-optimal for the dog health/well-being, because the radar signal enters too deeply into the skin. Moreover, the size of the antennae at these wavelengths can make the dog collar too large and can cause discomfort to the dog.

### Summary

One purpose of this disclosure is to improve the situation.

It is proposed an intelligent dog collar for monitoring physiological parameters of a dog, comprising:
- a movement sensor unit comprising an accelerometer and/or a gyrometer, wherein the movement sensor unit is configured to detect raw movement signals of the dog collar,
- a storage module storing a trained neural network, the neural network being configured to determine a physiologic information into raw movement signals detected by the movement sensor unit,
- a processing unit connected to the movement sensor unit and configured to operate the trained neural network,
- a memory configured to store the identified physiologic information,
- an interface for transmitting to an external communication device the identified physiologic information.

The trained neural network could be a deep neural network or DNN.

The following features can be optionally implemented, separately or in combination one with the others:
In general, the physiologic information may comprise/be a cardiac signal, a heart signal and/or a heart rate. Additionally, the physiologic information may further comprise a breathing signal and/or a breathing rate.

The radar unit is a millimeter wave (mmWave) radar unit.

In an embodiment, the frequency of the detecting signal is comprised between 57 GHz and 63 GHz. For instance, the radar unit is a radar from Infineon ^{®}.

In another embodiment, the frequency of the detecting signal is comprised between 76 GHz and 81 GHz. For instance, the radar unit is a radar from Texas Instrument ^{®}.

In an embodiment, the bandwidth of the detecting signal is 5 GHz.

In general, an input of the NN may comprise more than the raw RF signal. Namely, the input may comprise both the raw RF signal or an intermediate signal extracted thereof, and a raw movement signal.

In an embodiment, the dog collar further comprises a transmitting antenna adapted to emit a radiofrequency (RF) detecting signal having a frequency comprised between 57 GHz and 81 GHz, and at least a receiving antenna adapted to detect a raw RF signal which comprises reflection of the RF detecting signal, wherein the neural network is further configured to determine the physiologic information into raw movement signals thanks to the raw RF signals.

In general, the input of the NN may comprise any combination of the input signal list consisting in, for N antennae of the radar unit and M axis of the accelerometer and/or gyrometer: an N-dimensional raw radar In-phase signal, an N-dimensional distance signal, an N-dimensional velocity signal, an N-dimensional phase signal, one distance signal calculated by beamforming, one velocity signal calculated by beamforming, an M-dimensional acceleration signal, an M-dimensional angular velocity signal, one acceleration magnitude signal, one angular velocity magnitude signal.

The neural network comprises layers of calculating functions.

In an embodiment, the neural network performs a regression function, and the physiologic information comprises a heart rate (HR) of the dog or a breathing rate (BR) of the dog. Indeed, the raw RF signal comprises both the heart and the breathing information in a mixed way, which need to be discriminated from each other inside the raw RF signal. The use of the regression function enables a direct extraction of the heart rate and/or breathing rate features from sampled sensed signals. An advantage is that un-necessarily calculation of intermediate processing of the sampled signals are avoided, that is to say, the calculation cost is low. Further, the calculation can directly use software-accelerated or hardware-accelerated neural network inference functions. For instance, an output layer of the neural network performs a software function.

In an embodiment, the neural network has an encoder-decoder architecture, and is trained to output the physiologic information, wherein the physiologic information comprises the heart signal (HS) and/or the breathing signal (BS) and/or a heart peak probability signal and/or a breathing peak probability signal.

For instance, the neural network performs a denoising, and the physiologic information comprises a heart signal (HS).

For instance, the neural network performs a source separation, and the physiologic information comprises both a heart signal (HS) of the dog and a breathing signal (BS) of the dog.

For instance, the neural network performs a domain translation, and the physiologic information comprises a breathing signal (BS).

For instance, the neural network performs a segmentation, and the physiologic information comprises a heart peak probability signal of the dog and/or a breathing peak probability signal of the dog.

For instance, the neural network performs a segmentation function, and the physiologic information comprises all of a heart signal (HS) of the dog, a breathing signal (BS) of the dog, a heart peak probability function of the dog and a breathing peak probability signal of the dog. The heart peak probability signal is a numerical signal in which each numerical value represents a probability that a corresponding sample of an input of the NN is a peak of the heart signal. The input of the NN namely comprise raw RF signal.

Indeed, the raw RF signal comprises both the heart and the breathing information in a mixed way, which need to be discriminated from each other inside the raw RF signal.

The use of the segmentation function enables to calculate in a very efficient way a processed heart signal from sampled sensed signals. Hence, one can keep track of time-variations of the heart component of the sampled signals. In other words, a high level of information is outputted.

In an embodiment, the neural network is a U-NET neural network. Thanks to the use of a U-NET neural network, the segmentation is more accurate, as the network propagates context information to higher resolution layers.

In an embodiment, the neural network is a multi-task neural network, the neural network (NN) comprising a backbone of shared layers and two heads of task-specific layers, one of the head being the regression function and the other head being the segmentation function or the translation function. More heads are possible. For instance, the NN has three heads: one for a segmentation function, one for a regression function and one for a translation function.

In an embodiment, the interface is further configured for receiving an update of the neural network, and wherein the dog collar is further configured for storing the update into the storage module. Indeed, the dog collar being part of a fleet of dog collars, data collected in use by the fleet of dog collar constitute a growing provision for training data to be used in order to improve the training of the neural network, until an improved new version of the neural network is available. Continuous improving of the neural network may thus be possible according to the data collect thought the fleet. A variety of customized neural networks can be further trained from the neural network, or either trained from scratch as described below, for a special population of dogs. For instance, the special population of dogs is a population of dogs of the same breed. For instance, the customized neural network is further trained for a dedicated individual dog.

The invention also provides a distributed system comprising a fleet of dog collars as described herein above, a cloud application and a data repository configured to store raw movement signals and physiologic information collected from the fleet.

The invention also provides a method for providing a dog collar, the method comprising:
- providing a training collar comprising a movement sensor unit comprising an accelerometer and/or a gyrometer, wherein the movement sensor unit is configured to detect raw movement signals of the dog collar,
- providing a training device adapted to be worn by a dog on a body part of the dog and configured to detect a heartbeat reference signal from the dog,
- training a neural network, wherein the training comprises:
   the acquisition of training data, comprising, for each dog of a population of dogs,
   * detecting a raw movement signal segment and a heartbeat reference signal segment during the simultaneous wearing, by the dog, of both the training collar and the training device,
   * determining peaks in the heartbeat reference signal segment and labeling said peaks in the heartbeat reference signal segments,
   * storing the raw movement signal segment in association with the heartbeat reference signal segment and the labeled peaks,
   * feeding the neural network with said training data, in order to train the neural network for a task, wherein the task comprises an determination of a physiologic information from raw movement signals detected by the movement sensor unit,
- recording, in a storage module, the trained neural network (NN),
- and providing a dog collar comprising a same movement sensor unit as the movement sensor unit of the training collar, and the storage module comprising the trained neural network (NN).

In an embodiment, the training device is adapted to be worn by a dog on a different body part of the dog than around the neck, and the training device is configured to detect a heartbeat reference signal from another part of the dog than the throat,

In an embodiment, the training further comprises :
- determining an activity state in the raw movement signal segment and labeling said activity state in the raw movement signal segment, the activity state comprising at least a rest state of the dog,
- storing the raw movement signal segment in association with the labeled activity state,
the training data further comprising said raw movement signal segment and labeled activity state, the method further comprising training the neural network to determine a rest state of the dog into raw movement signals detected by the movement sensor unit (12).

In an embodiment, the body part of the dog is the ear, the groin, or the chest.

Thanks to these features, the heartbeat reference signal is not prevented from being detected by the hairs. Indeed, the groin is located between the hip and the thigh , which is a low-density hair area.

There are various kinds of training devices that can be used in order to get a heartbeat reference signal. The training device comprises sensors configured for detecting the heartbeat reference signal.

For instance, the training device may be an optical receiver such as for instance a camera or an infrared detector. The heartbeat reference signal may be a photoplethysmogram (PPG).

In another example, the training device may comprise an electrical sensor, and the heartbeat reference signal may be a heart impulse signal or an electrocardiogram (ECG) signal. In another example, the training device may comprise a second radar unit and the body part is above the femoral artery of a back leg.

Great care must be taken to ensure synchronous data acquisition of the sensor of the training device and the radar unit of the training dog collar. To this end, the training device may be connected to the training collar either with an electric wire or through a radio protocol like bluetooth, so that the collar can record the data streams altogether in synchronization.

The following features can be optionally implemented, separately or in combination one with the others:
- The reduced amount of labels obtained from the heartbeat reference signals may be overcome by the use of self-supervision, semi-supervised domain adaptation or transfert learning from training done on simulated data. In an advantageous embodiment, the labeling of said peaks in the heartbeat reference signal segments is automatically generated by the neural network, such that the neural network is self-supervised.
- the training device comprises an optical sensor and the heartbeat reference signal is a photoplethysmogram (PPG).
- the training device is a pulse oximeter.
- the training device is an ECG sensor. The heartbeat reference signal is an ECG. Such a variant is very advantageous, because the ECG comprises both peaks of the heart and peaks of the breathing. It is therefore possible to train the neural network by means of labelling the peaks of both the heart and the breathing.
- the dog collar further comprises a transmitting antenna adapted to emit a radiofrequency (RF) detecting signal having a frequency comprised between 57 GHz and 81 GHz, and at least a receiving antenna adapted to detect a raw RF signal which comprises reflection of the RF detecting signal, wherein the training further comprises: - detecting a raw RF signal segment during said simultaneously wearing;- storing the RF signal segment in association with the raw movement signal the heartbeat reference signal segment and the labeled peaks.
- the raw RF signal is pre-processed to extract the displacement (d) and/or velocity of the skin and/or hairs of the throat. The pre-processing may comprise the previous extraction of the phase of raw RF signal.
- the raw RF signal is pre-processed to extract the velocity (v) of the skin and/or hairs of the throat. The pre-processing may comprise the previous extraction of the phase of raw RF signal.
- the trained task comprises a regression function, and the physiologic information comprises the heart rate.
- the neural network has an encoder-decoder architecture, and is trained to output the physiologic information, wherein the physiologic information comprises the heart signal (HS) or the breathing signal (BS) or a heart peak probability signal or a breathing peak probability signal,
- the trained task comprises a translation function, and the physiologic information comprises the heart signal.
- the trained task comprises a segmentation function, and the physiologic information comprises a heart peak probability signal,
- the training further comprises the simulation of further training data, wherein the simulation comprises: providing a simulated heartbeat reference signal and providing a simulated raw RF signal from the simulated heartbeat reference signal;
- the intelligent dog collar does not comprise a radar unit;
- sensing units of the intelligent dog collar does not comprise a radar unit,
- sensing units of the intelligent dog collar comprise only the movement sensor unit,
- sensing units of the intelligent dog collar consist in the movement sensor unit,
- the neural network is configured to determine a physiologic information into signals that comprise the raw movement signal, and do not comprise raw RF signals;
- the neural network is configured to determine a physiologic information into signals comprising only the raw movement signals;
- the input of the trained neural network consists in said raw movement signals;

### Brief Description of Drawings

Other features, details and advantages will be shown in the following detailed description and on the figures, on which:
**Fig. 1**

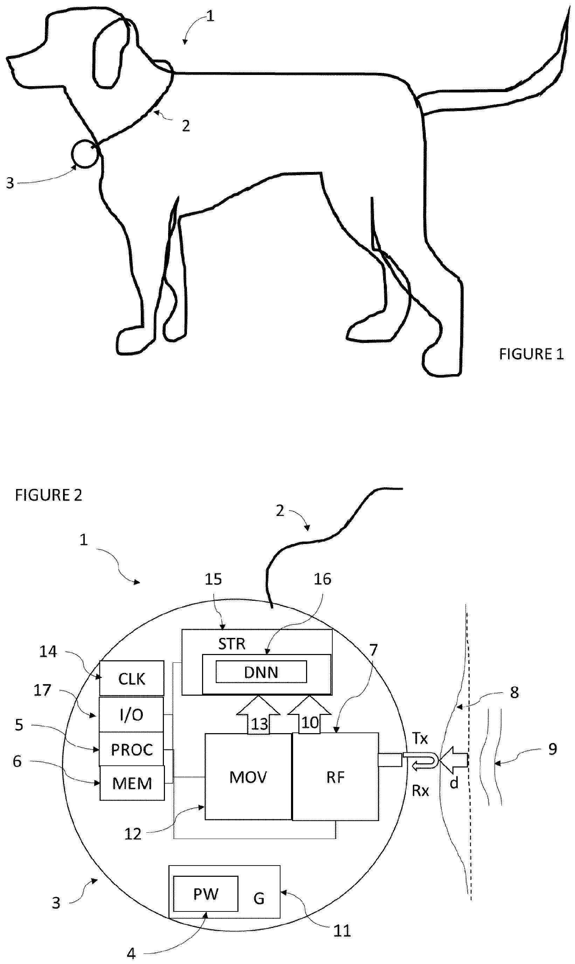

   [Fig. 1] is an illustration of a dog wearing a dog collar according to the invention.
**Fig. 2**
   [Fig. 2] is an illustration of the dog collar of Figure 1.
**Fig. 3**

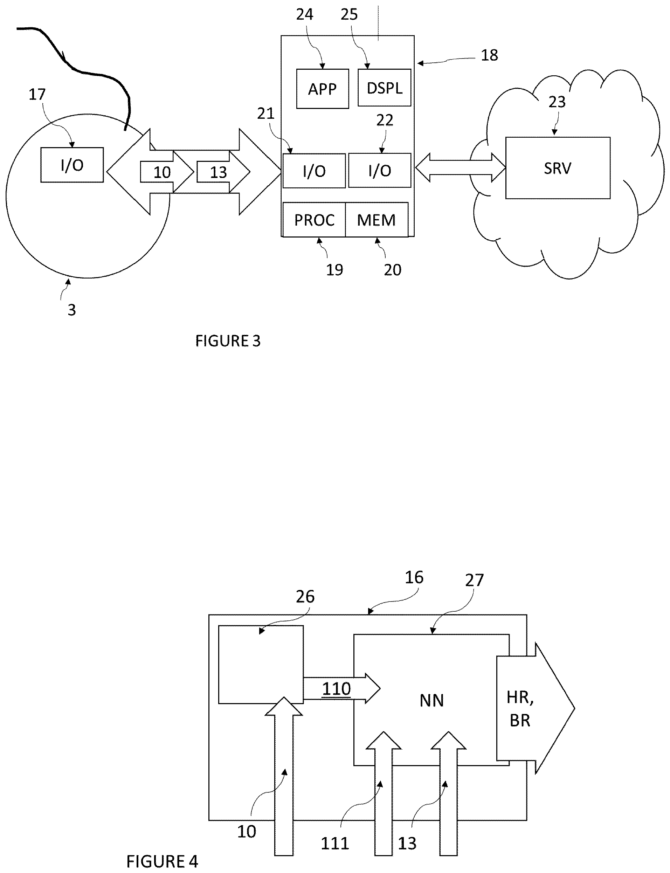

   [Fig. 3] is an illustration of a communication system comprising the dog collar of Figure 2.
**Fig. 4**
   [Fig. 4] is an illustration of a neural network comprised in the dog collar of Figure 2.
**Fig. 5**

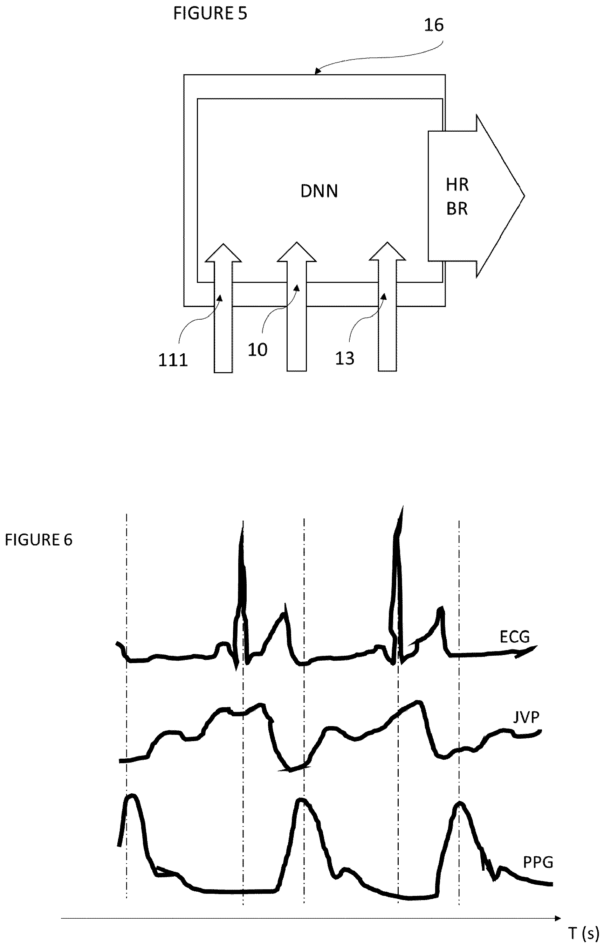

   [Fig. 5] is an illustration of a variant of the neural network of Figure 4.
**Fig. 6**
   [Fig. 6] is an illustration of synchronous electrocardiogram (ECG) signal, raw RF signal and PPG signal, for the sake of comparison.
**Fig. 7**

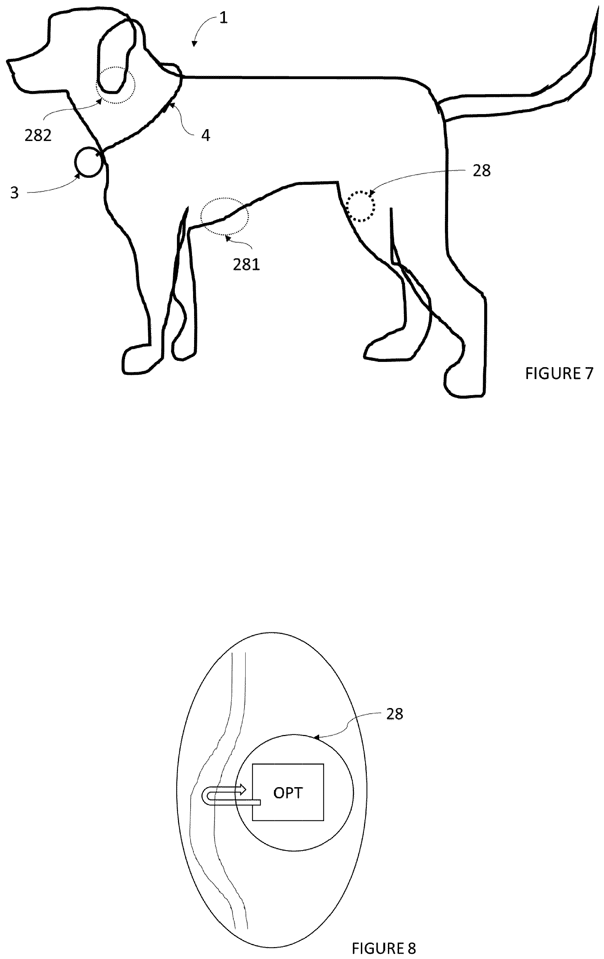

   [Fig.7]. is an illustration of a dog wearing a training dog collar similar to the dog collar of Figure 2 and a supplementary training device which can be arranged on three different locations on the dog.
**Fig. 8**
   [Fig. 8] is an illustration of the supplementary training device.
**Fig. 9**

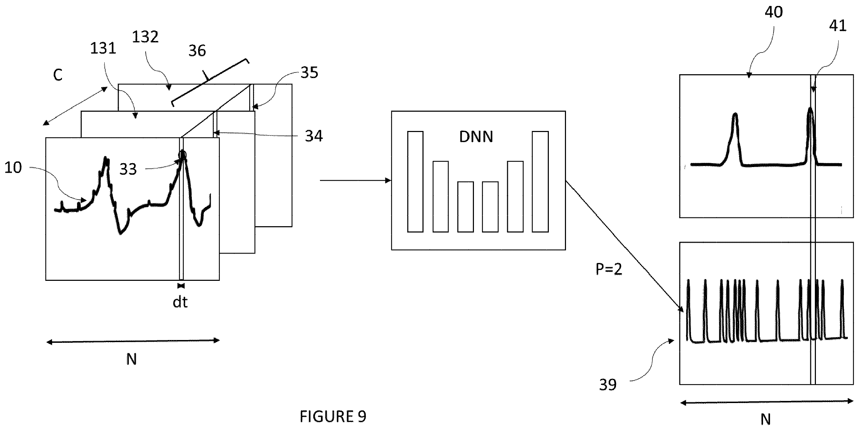

   [Fig. 9] is an illustration of an example of a encoder-decoder-type neural network embedded in the dog collar of Figure 1 in an inference phase.
**Fig. 10**

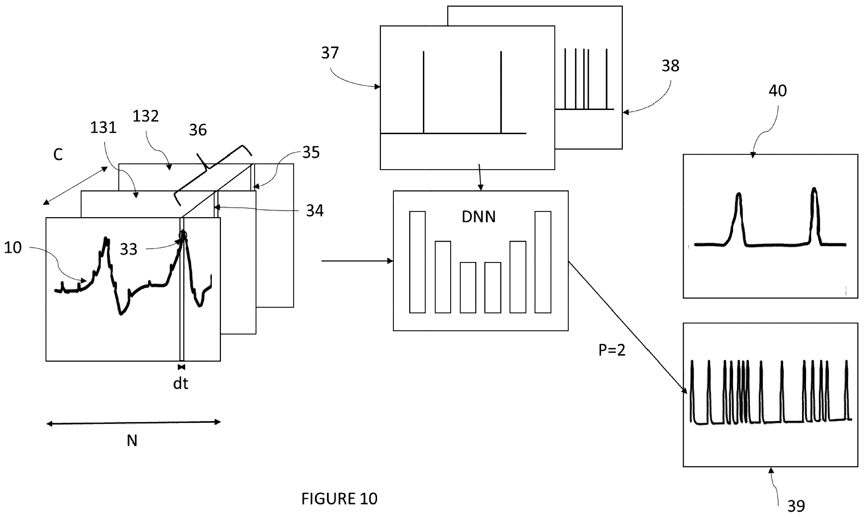

   [Fig. 10] is an illustration of an example of the encoder-decoder-type neural network of Figure 9 in a training phase.
**Fig. 11**

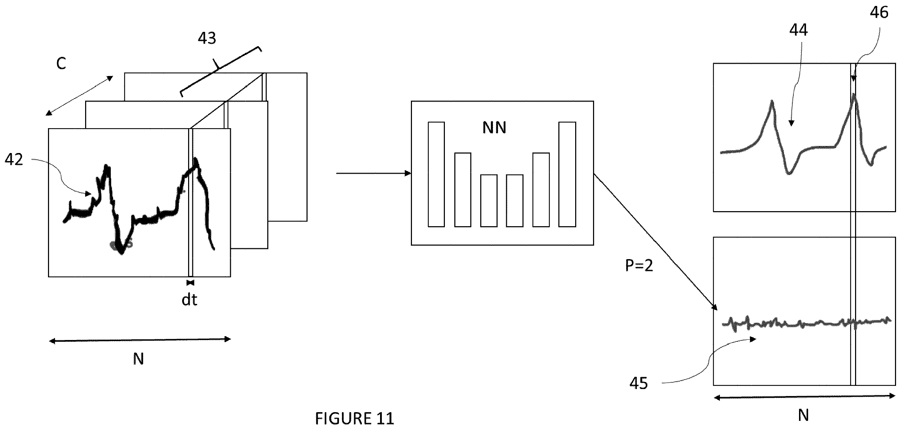

   [Fig. 11] is an illustration of an example of a source separation-type neural network embedded in the dog collar of Figure 1 in an inference phase.
**Fig. 12**

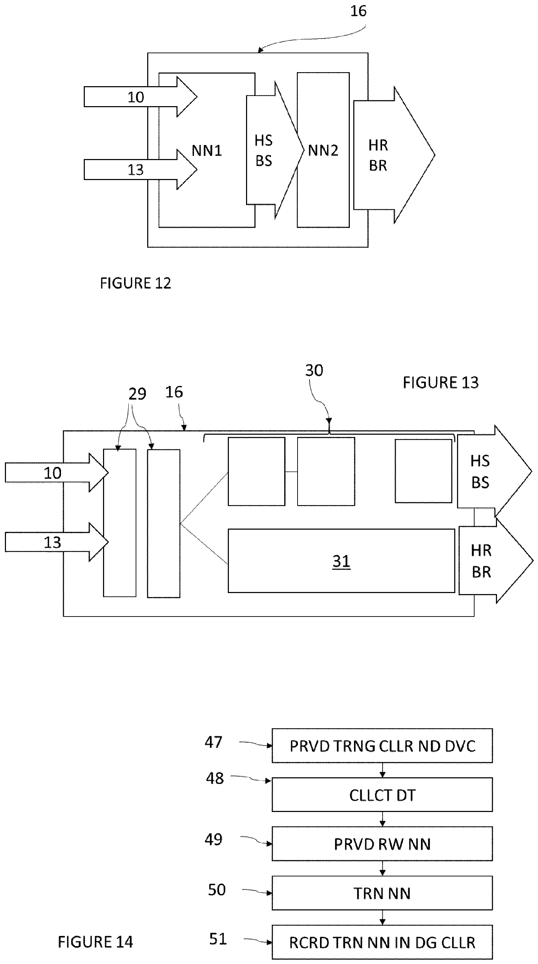

   [Fig. 12] is an illustration of another variant of the neural network of Figure 4.
**Fig. 13**
   [Fig. 13] is an illustration of another variant of the neural network of Figure 4 wherein the neural network is a multi-task one.
**Fig. 14**
   [Fig. 14] is an illustration of a method for providing the dog collar of Figure 1.

### Description of Embodiments

Figures and the following detailed description contain, essentially, some exact elements. They can be used to enhance understanding the disclosure and, also, to define the invention if necessary.

### Intelligent dog collar

One can see on **Figure 1** and **Figure 2** an intelligent dog collar 1 for monitoring physiological parameters of a dog.

As one can see, **the dog collar 1** comprises a band 2 for being fitted around the neck of a dog, and a set of electronic parts 3 which is adapted on the band 2 for monitoring physiological parameters of a dog. On the figures, the electronic parts 3 are represented in a unitary manner, for instance the electronic parts 3 are arranged in a single medallion. However, in some variants, the electronic parts 3 may be distributed on the band 2 in any manner.

The dog collar 1 comprises a power source 4, such as a rechargeable battery / solar panels / etc., configured for electrically powering the electronic parts 3.

The electronic parts 3 comprise a processing unit 5 and a memory 6.

In an embodiment, the electronic parts 3 comprise **a radar unit 7.** The radar unit 7 comprises a transmitting antenna(s) adapted to emit a radiofrequency (RF) emitting signal Tx at 60GHz with a 5.5GHz bandwidth, for instance. The radar unit 7 further comprises a sensor comprising at least a receiving antenna adapted to receive reflections Rx of the RF detecting signal Tx on the skin 8, above the throat of the dog for instance, preferably above the jugular vein 9 and/or carotid vein of the dog, such that the radar unit 7 is configured to detect a raw RF signal 10 from said reflections Rx. The raw RF signal 10 recorded and/or extracted from the radar unit 7 may be a unidimensional signal. For instance, the raw RF signal 10 is measured by a single antenna. In another example, the sensor of the radar unit 7 comprises three antennas. In this case, the raw RF signal 10 is a 3-dimension signal, wherein each dimension is measured by a different antenna.

For instance, the sampling rate of the raw RF signal 10, also referred to as a frame- rate is comprised in the range [50-200Hz].

The radiofrequency (RF) detecting signal Tx at 60GHz is safe for the health of the dog, because at this frequency, the wave does not deeply enter the skin. Additionally, the size of the antennas is small for the frequency, so the radar unit 7 is easy to embed into the dog collar 1.

In an embodiment, the radar unit 7 comprises a preventing module for preventing the transmitting and receiving antennas to be too close to the skin of the dog. The preventing module may for instance comprise a foam which is transparent for the frequencies around 60GHz. For instance, the preventing module may have a hollow shape which keeps the transmitting and receiving antennas at a predetermined distance from the skin of the dog. Such a preventing module has the advantages of preventing the flattening of the jugular vein and/or dog hair and of increasing the detection of skin area seen by the radar unit 7.

Advantageously, the dog collar comprises a ballast 11 for enabling a positioning of the radar unit 7 on the jugular vein. For instance, the ballast 11 comprises the power source 4.

In an embodiment, the electronic parts 3 comprise a **movement sensor unit 12.** The movement sensor unit 12 comprises a motion sensor comprising an accelerometer and/or a gyrometer. In a number of embodiments of the invention, the movement sensor unit may further comprise further motion sensors such as for example a magnetometer. The movement sensor unit 12 is configured to detect raw movement signals 13 of the dog collar 1.

A raw movement signal 13 recorded and/or extracted from the movement sensor unit 12 may be a unidimensional signal.

For instance, the raw movement signal 13 is measured by a single-axis accelerometer. In another example, the sensor of the movement sensor unit 12 is a multi-axis accelerometer, for instance a three-axis accelerometer. The raw movement signal 13 is therefore a 3-dimensional signal, wherein each dimension corresponds to one of the three-axis.

In another example, the movement sensor unit 12 comprises a gyrometer. The raw movement signal 13 then comprises a measured angular velocity.

In another example, the movement sensor unit 12 comprises a gyrometer and a single-axis accelerometer, and the raw movement signal 13 is a 2-dimensional signal, wherein a first dimension corresponds to a measured acceleration and a second dimension corresponds to a measured angular velocity.

In another example, the movement sensor unit 12 comprises a gyrometer and a three-axis accelerometer, and the raw movement signal 13 is a 4-dimensional signal, wherein a first dimension corresponds to a measured angular velocity and each of the three other dimensions corresponds to the acceleration measured for one of the three-axis.

It is worth noting that, although both a movement sensor unit and a radar unit are represented in figure 2, in a number of embodiments of the invention, the dog collar comprises only one of the two units.

For example, in an embodiment, the dog collar comprises a movement sensor unit, but does not comprise a radar unit.

In an embodiment, the dog collar does not comprise a movement sensor unit, but comprises a radar unit.

In an embodiment, the dog collar comprises both a movement sensor unit, and a radar unit.

More generally, the dog collar may comprise one or more sensing units, a sensing unit being a unit dedicated to the measurement of physical parameters of the collar or its environment. For example, even if they both comprise antenna, the radar unit 7 is a sensing unit, because it aims at sensing the environment of the collar while the short-range radio communication interface 17 is not a sensing unit, because it aims at establishing a communication rather than performing measurement of physical parameters of the collar or its environment.

Therefore, in a number of embodiments of the invention, the dog collar comprises one or more sensing units, and:
- in a number of embodiments, the one or more sensing units comprise a movement sensor unit, but does not comprise a radar unit;
- in a number of embodiments, the one or more sensing units comprise a radar unit, but does not comprise a movement sensing unit;
- in a number of embodiments, the one or more sensing units comprise both a radar unit, and a movement sensing unit;
- in a number of embodiments, the one or more sensing units comprise, in addition of a radar and/or a movement sensing unit, further sensing units. Such further sensing units may typically comprise further sensors which are different from the radar and movement sensors.

The dog collar 1 further comprises a clock unit 14 configured to synchronize a segment of the raw RF signal 10 with a segment of raw movement signal 13.

The dog collar 1 further comprises a storage module 15 storing a **neural network unit 16** comprising an artificial intelligence (Al) software. The Al software comprises at least a trained neural network (NN).

In general, a NN is a computing system which is inspired by the biological neural networks that constitute animal brains. Namely, the NN comprises a collection of connected nodes organized in layers. Each node is configured for receiving inputs, which are numerical values, from nodes of the previous layer, processing the inputs by a nonlinear function, and outputting the processed inputs for the connected nodes of the following layer.

A raw NN has untrained nodes, that is to say, the coefficients of the nonlinear functions in the nodes are raw/random coefficients.

The raw NN is prior trained in order to become a trained NN. A trained NN is typically configured for executing a task, wherein the task is the extraction, from at least an input received by the NN in nominal use, of an output. Such an extraction is commonly referred to as an "inference" of the NN.

In other word, in a prior training phase, the raw NN is configured. In an inference phase, the trained NN is executed.

The training comprises the learning of the task.

In the case where the learning is a supervised learning, the training typically implies:
- collecting training inputs usable as inputs for the raw NN,
- defining a training output corresponding to a training input,
- labeling the training input with a label representing the corresponding training output,
- inputting into the raw NN the labeled training inputs, such that the raw coefficients are modified until the calculated outputs converge to the training outputs. The raw coefficients are commonly referred to as "weights" in the neural network literature.
- storing the trained NN having the modified coefficients.

In the case where the learning is a semi-supervised learning, only part of the training inputs needs to be labeled.

In the case where the learning is a self-supervised learning, the NN is trained on the same data but for another task of generation of label for the training inputs. This label generation task can be used to pretrain the NN (tasks in series) or can be learned together with the main supervised task in a multi-task manner (tasks in parallel).

Another self-supervised NN may be used, in which some training data may be pre-labelled for unassigned tasks.

In a number of embodiments of the invention, the neural network unit 16 is configured to determine **a physiologic information** from the segment of the raw RF signals 10 and/or the segment of the raw movement signals 13. In other words, a task learned by the NN is the determination of the physiologic information, the input of the NN comprises the segment of the raw RF signals 10 and/or the segment of the raw movement signals 13, and the output of the NN comprises the physiologic information.

For example:
- in a number of embodiments, the input of the NN comprises the raw RF signals 10, but does not comprise the raw movement signals 13;
- in a number of embodiments, the input of the NN comprises the raw movement signals 13, but does not comprise the raw RF signals 10; the input of the NN may consist in the raw movement signals 13, and/or comprise only raw movement signals 13;
- in a number of embodiments, the input of the NN comprises both the raw movement signals 13, and the raw RF signals 10;
- in a number of embodiments, the input of the NN further comprises, in addition to the raw movement signals 13, and/or the raw RF signals 10, the signals outputted by further sensing units.

The physiologic information may comprise various information. Namely, the physiologic information comprises a heart signal (**HS**) and/ or a heart rate (**HR**).

In some embodiments, the physiologic information may further comprise a breathing signal (**BS**) and/or a breathing rate (**BR**).

The processing unit 5 connects the other electronic parts 3 and is configured to operate the neural network unit 16.

The memory 6 is configured to store the identified physiologic information in relation with of the segment of the raw RF signal 10 and the segment of the raw movement signal 13 from which the physiologic information is extracted.

The dog collar 1 may also comprise a short-range radio communication interface 17 allowing the communication of data between the dog collar 1 and a mobile device 18 distinct from the dog collar 1, as represented on **Figure 3****.** In one embodiment, the short-range radio communication interface is using a Wi-Fi, Bluetooth^{®}, LORA^{®}, SigFox^{®} or NBIoT network. In another embodiment, it can also communicate using a 2G, 3G, 4G or 5G network.

Namely, the short-range radio communication interface 17 is configured to send messages comprising recent physiologic information in association with the segment of the raw RF signal 10 and/or the segment of the raw movement signal 13 from which the physiologic information is extracted. For example, the messages can be push-messages sent on a periodic manner. In variant, the messages can be response messages sent in response to receive a message request from the mobile device 18 for update. In alternative, the sending is performed after a predetermined movement detected by the movement sensor unit 12 (e.g. after a run, if it is determined that the run is a moment of interest for analyzing raw data). The data can also be sent if the battery level is below a predetermined value, in order to avoid any loss of data.

The mobile device 18 can typically be an electronic tablet, a mobile phone or a computer.

The mobile device 18 comprises a processing unit 19 and a memory 20 as well as a short-range radio communication interface 21 enabling communication with the dog collar 1. The mobile device 18 further comprises a communication interface 22 enabling communication with a distance server 23 in a known manner.

The mobile device 1 further comprises an application module 24 configured to run an application for monitoring physiological parameters of a dog. For instance, the application module 24 is configured to extract a recommendation to visit the veterinarian from the physiologic information. For instance, the application module 23 is configured to display the physiologic information on a screen 25 of the mobile device 18. Thus, a dog owner may access relevant health information about her/his dog wearing the dog collar 1 by using her/his mobile device 18.

A fleet of dog collars 1 may be connected to the distance server 23 through mobile devices 18 of the dog owners. Each dog collar 1 of the fleet is identified by a dog collar identifier.

The fleet of dog collars 1 can collect fleet data from the population of dogs wearing the dog collars 1. Namely, the fleet data comprise, in association with the dog collar identifier, the physiologic information in association with the segment of the raw RF signal 10 and/or the segment of the raw movement signal 13 from which the physiologic information is extracted.

Thus, the distance server 23 can store the fleet data.

The fleet data constitute a provision for training data to be used in order to train the neural network unit 16 for various tasks. As time goes by, the fleet data grows and the neural network unit 16 can be further trained by new data in order to be improved, such that a new version of the neural network unit 16 is available.

Thus, the new version of the neural network unit 16 can be updated in the fleet of dog collars 1 by loading from the distance server 23 through the mobile devices 18.

In an embodiment, the neural network unit 16 can be further fine-tuned. For instance, the neural network unit 16 can be retrained with new training data classified according to different breeds of dogs, or even individualized.

### Collection of initial training data.

To train the neural network unit 16 from scratch, initial training data should be collected from an initial set of dogs and recorded into the distance server 23.

In order to do so, a training collar is used on the initial set of dogs.

The training collar is similar to the dog collar 1 above described. Advantageously, the training collar is configured in order to record measured raw RF signal from its radar unit 7 and/or the segment of the raw movement signal 13 from its movement sensor unit 12 into the memory. For instance, the memory is a Storage Device (SD) card. In such a case, one can record a lot of raw RF signals 10 for use in the training phase of the NN.

The initial training data comprises either raw RF signals, or intermediate signal extracted therefrom.

### Feature extraction

As represented on **Figure 4****,** the neural network unit 16 comprises a feature extraction unit 26 being configured to extract at least an intermediate signal from the raw RF signal and/or the raw movement signal 13. An example of intermediate signal is the distance to the skin computed from one Rx antenna of the radar unit 7. Therefore, the intermediate signal comprises at least the feature of the displacement d of the skin above the jugular vein.

Such a displacement over the time can be interpreted as a jugular vein pulse (JVP) signal.

In a **first example,** the radar unit 7 comprises a Continuous Wave (CW) radar and the detecting signal Tx is a Continuous Wave (CW) signal. The raw RF signal 10 comprises the CW signal and the intermediate signal is calculated as follows.

In the first example, the calculated phase PHI is calculated by applying a function to the detecting signal Tx and the reflections Rx. The function is such that PHI = arctan (Q/I), wherein. Q and I represent the quadrature and in-phase signals derived from the mixing of TX and RX signals.

Then, the displacement d of the skin is extracted from the calculated phase PHI. Indeed, there is a relationship between a calculated phase PHI and the displacement d of the skin. The relationship between the calculated phase PHI and the displacement of the skin is the following: PHI = 2 pi *d/ lambda, wherein lambda represents the wavelength of the detecting signal Tx.

In such a case, the intermediate signal therefore comprises the calculated distance to the skin, which is a relative distance. An advantage of such a CW radar is that its cost is less expensive than other kind of radars.

In a **second example,** the radar unit 7 comprises a Frequency Modulated Continuous Wave (FMCW) radar. The intermediate signals may be extracted according to a first extracting mode or a second extracting mode from the detecting signal Tx, which is a FMCW signal. The raw RF signals 10 comprises the FMCW signal.

In the second example, the calculated phase PHI is the arctan function of the imaginary part over the real part of the interest Fourier coefficient of the Fast Fourier Transform Range-FFT. In some configurations, one degree for the phase PHI can correspond to a displacement d of roughly 1 µm.

In the **first extracting mode,** the radar unit 7 is configured to emit one chirp per radar-frame. In this case, the intermediate signal comprises the displacement d of the skin above the jugular vein.

In the **second extracting mode,** the radar unit 7 is configured to emit multiple chirps per radar-frame. In other words, the radar unit 7 is configured to emit bursts of chirps. In this case, the intermediate signals comprise the displacement d of the skin above the jugular vein, and the velocity of said skin which is deduced from the Doppler-FFT.

The second extracting mode is more energy consumptive than the first extracting mode because the number of chirps is higher. In order to reduce the required number of chirps per radar-frame in the detecting signal Tx, the feature extraction unit 26 may be further configured to select the first extracting mode or the second extracting mode. For instance, the selection is performed depending on the quality requirements.

In a **third example,** the radar unit 7 comprises at least a number N of receiving antennas adapted to receive reflections Rx of the RF detecting signal Tx. For instance, the radar unit 7 comprises a number P of transmitting antennas Tx.

In this case, the raw RF signal 10 is a N-dimension signal, wherein each dimension is measured by a pair of antennae comprising one emission antenna and one reception antenna.

In such an example, the feature extraction unit 26 may be configured for performing a beamforming function on the raw RF signal 10, in order to extract the intermediate signal. The beamforming function is an algorithm which receives in inputs the different dimensions of the raw RF signal. The algorithm adapts filtering functions for each dimension before adding them together, in order to optimize a parameter.

In the third example, the beamforming is performed such that that the output of the beamforming is an intermediate signal with the higher signal/ noise ratio (SNR). In other words, the different dimensions are processed all together in order to destruct the noise seen by the antennas in the calculated intermediate signal. In other words, the parameter which is optimized is the SNR.

In a **fourth example,** the parameter which is optimized is the periodicity of the calculated intermediate signal. In other words, the algorithm does not optimize the amplitude but the periodicity.

In a **fifth example,** the feature extraction unit 26 may be configured for performing two beamforming functions on the raw RF signal 10. The first beamforming function optimizes the breathing periodicity of the intermediate signal. The second beamforming function optimizes the heartbeat periodicity of the intermediate signal.

The above five examples may be cumulated in order to further improve the intermediate signal. For instance, the feature extraction unit 26 may comprise both a feature extraction from the FMCWsignal and a beamforming function.

### Training phase - training data

The neural network unit 16 further comprises a calculation unit 27 comprising at least a neural network (NN).

In the example depicted on **Figure 4****,** the feature extraction unit 26 may be a handcrafted algorithm or an artificial intelligence software. In another embodiment, the feature extraction unit 26 may comprise a neural network NN. In this case, the initial training data comprises the intermediate signals 110 extracted from the raw RF signal 10 and/or the raw movement signal 13 as described hereinabove.

By contrast, **Figure 5** represents a variant of the neural network unit 16 wherein the feature extraction unit 26 and the calculation unit 27 are implemented as a single end-to-end NN. In the variant, the NN is directly fed by the initial training data.

In both cases, the initial training data comprise the raw RF signal 10 segments, and/or the raw movement signals 13. The initial training data may optionally comprise signals outputted by further sensing units.

For example:
- in a number of embodiments, the initial training data comprises the raw RF signals 10, but does not comprise the raw movement signals 13;
- in a number of embodiments, the initial training data comprises the raw movement signals 13, but does not comprise the raw RF signals 10; the initial training data may consist in the raw movement signals 13, and/or comprise only raw movement signals 13;
- in a number of embodiments, the initial training data comprises both the raw movement signals 13, and the raw RF signals 10;
- in a number of embodiments, the initial training data further comprises, in addition to the raw movement signals 13, and/or the raw RF signals 10, the signals outputted by further sensing units.

In both cases, the NN is trained to determine the physiologic information from the initial training data until the NN converges.

In some cases, the intermediate signals extracted from the raw RF signal comprise mixed information and noise.

Indeed, the displacement d of the skin contains both the respiration between 0.1 and 0.5 Hz and the heart between 0.8 and 2 Hz. Moreover, the heart has a period that varies quickly because of the sinus arrhythmia in the dog population.

Moreover, in some cases, the dog collar 1 may not have a precise position around the neck, for instance when the dog collar 1 is provided to adapt to dogs of different morphologies. Therefore, the radar unit 7 and/or movement sensing unit 13 may have a non-optimal position above the throat. Depending on the position of the collar, the amplitudes of respiratory and physiologic movements may vary significantly. Therefore, distinguishing between breathing and heart is hard.

In fact, the displacement d of the skin results from various grounds, which namely comprise:
- the carotid pulses,
- the jugular pulses,
- the breathing pulse,
- the relative motion of the dog collar 1 compared to the dog wearing the dog collar 1, and
- some noise.

Therefore, as represented on **Figure 6****,** one can see that the intermediate signals comprising the JVP signal are not as easy to interpret the heart pulses as a Photoplethysmography (PPG) or into an Electrocardiogram (ECG).

In order that the NN may converge and better interpret the JVP signal, that is to say, in order to get the physiologic information from the JVP signal, a heartbeat reference signal 111 is used to be crosschecked with the JVP signal.

One can directly extract a heart signal HS or a heart rate HR from the heartbeat reference signals 111. The initial training data further comprise the heartbeat reference signal 111.

In order to acquire the heartbeat reference signal 111, one can use a training device 28 on the dog simultaneously wearing the dog collar 1.

For instance, the training device 28 is a pulse oximeter and the heartbeat reference signal 111 is a PPG.

The training device 28 may be arranged on the groin during the initial training data collection. Indeed, the groin is an area wherein the body hair is rare, so the signals are less disrupted. In such a case, the approach is reflective pulse oximetry. Such a training device 28 is represented with reference to the **Figures 7 and 8****.**

In variant, the training device 28 may be arranged on the ear, as represented on Figure 6 on the location 282. In such case, one can use transmissive pulse oximetry.

In an advantageous example, the training device 28 is an electrocardiogram sensor and the heartbeat reference signal is an ECG. For instance, the electrocardiogram sensor may be located on any location on the dog, such as for instance on the chest, as represented on Figure 6 on the location 281.

The training device 28 is configured to send to the distance server 23 the collected heartbeat reference signal segments synchronized with the raw RF signal segments collected at the same time by the radar unit 7 and/or the raw movement signals collected by the movement sensor unit 12 of the dog collar 1.

The initial training data may further comprise further signals collected by further sensing units.

The initial training data may further comprise displacement signals from which one can extract a different part of the displacement d of the skin due to a different ground.

Namely, to remove the part of the displacement d due to the relative motion of the dog collar 1, one can cross-check the JVP signal with the raw movement signal 13.

In such a case, the NN is fed by the intermediate signal 110 and by the raw movement signal 13 for training until the task of determination of the physiologic information from the intermediate signal is known. In other words, the NN performs a sensor fusion. The inputs of the NN are heterogeneous data from different sensors considered as a single big input.

In general, the initial training data may be sparse. Different options may be contemplated remedying to the lack of initial training data:
- using a semi-supervised learning and a transfer, also referred to as a domain adaptation from a target domain to a source domain (for instance from HR or HS to raw RF signals),
- using a semi-supervised learning,
- simulating at least part of the initial training data,
- augmenting at least part of the initial training data by using time stretching, that is to say, by a resampling.

### Training phase - Different tasks

In general, the neural network unit 16 may be further trained for other tasks in addition to the first task of determination of the physiologic information from raw RF signal and/or raw movement signals or from intermediate signal.

For instance, the neural network unit 16 may be trained for a second task, which is the determination of an activity class of the dog wearing the dog collar 1 from the raw movement signal.

The learning therefore implied a supervised learning of the neural network unit 16 for the second task, wherein segments of the raw movement signal 13 are associated with labels representing the class. For instance, these labels correspond to whether or not the segment of the raw movement signal 13 is recorded at a moment wherein the dog is at rest.

For instance, the labels comprise the following classification: walking, running, barking, scratching, sleeping, at rest.

In an advantageous variant, the NN of the neural network unit 16 is trained to learn the second task and the first task together. Advantageously, a single calculation stream is shared for the first and the second task. Moreover, the first and the second task may be correlated. For instance, when the dog is running, the heart rate is expected to increase. Therefore, for instance, it is useful to detect that the dog runs (second task) in order to estimate its heart rate (first task).

### Kind of neural network

In general, the NN of the neural network unit 16 can be of various kinds: either using a regression NN, or an encoder-decoder NN.

In all cases, the NN of the neural network unit 16 is configured to execute at least a task, by receiving at least an input and by outputting at least the physiologic information. In an advantageous embodiment, the physiologic information is further labeled with the corresponding activity class. The following will describe a plurality of embodiments for the neural network unit 16.

In all the embodiments described below, the input of the NN may comprise any combination of the **input signal list** consisting in, for N antennae and M axis:
- an N-dimensional raw radar In-phase signal.
- an N-dimensional distance signal.
- an N-dimensional velocity signal,
- an N-dimensional phase signal,
- one distance signal calculated by beamforming,
- one velocity signal calculated by beamforming,
- an M-dimensional acceleration signal,
- an M-dimensional angular velocity signal,
- one acceleration magnitude signal,
- one angular velocity magnitude signal.

As one can see, in the embodiments described below the input signal list comprises the raw RF signal 10 or the intermediate signals extracted thereof. As described above, the selected number of antennas may be 3. The number of axis may be 3.

### Regression-type NN

A regression-type NN architecture may comprise convolutional neural networks (CNN), full CNN, long short-term memory (LSTM) neural networks, and/or transformers. Convolutional neural network possible architecture may be for instance ResNet, a RegNet or EfficientNet.

For instance, the inputs are segments of the raw RF signal 10 and/or of the raw movement signal 13 over the time. A segment is a numerical sampling of a part of the signal. A frame is an association of segments of different signals acquired at the same moment. For instance, the frame comprises two segments, a segment of the raw RF signal 10 and a segment of the raw movement signal 13. For instance, the part of the signal corresponds to a 30 sec duration, and the segment is sampled into 6000 numerical values.

For the regression-type NN, the physiologic information is either the instantaneous heart rate (HR) or the breathing rate (BR).

In a **first embodiment,** the NN of the neural network unit 16 executes an end-to-end regression in order to extract from the inputs the heart rate (HR). In other words, the output is the HR. In order to get the breathing rate (BR), the neural network unit 16 should execute a second end-to-end regression.

In a **second embodiment**, the NN of the neural network unit 16 executes an end-to-end regression in order to extract from the inputs both the heart rate (HR) and the breathing rate (BR). In other words, the neural network unit 16 has a two-dimensional output.

In the second embodiment, the training is performed as follows:
In a first step, training data is collected from a plurality of dogs. the training data comprises frames recorded from the various sensors (radar unit 7 and/or movement sensor unit 12 and/or further sensing units and training device 28) and comprising mixed information including the heart signal (HS), the breathing signal (BS), the movement and noise.

For instance, a frame comprises three segments: one of the raw RF signal 10 or the intermediate signals extracted thereof (displacement and/or velocity), one of the raw movement signal 13, and a heartbeat reference signal 111 from the training device 28. For instance, the heartbeat reference signal 111 is a PPG or ECG signal.

In a second step, for each input frame, the heart rate (HR) is calculated from the segment of the heartbeat reference signal. The heart rate is calculated by means of the determination of the peaks in the heartbeat reference signal, then calculation of a mean period between two peaks. In an advantageous embodiment, the heart rate (HR) is further calculated to discriminate between the diastole phase and the systole phase of the heart.

In a third step, for each input frame, the breathing rate (BR) can be calculated from classical algorithm such as FFT on the segment of the raw RF signal or from the segment of the heartbeat reference signal.

In a fourth step, the NN is trained for executing the regression to extract the heart rate (HR) and the breathing rate (BR) from the input of the NN. In the second embodiment, the input may comprise either the raw RF signal 10 or the intermediate signals 110. The input may further comprise the raw movement signals 13.

Optionally, for each input frame, a confidence indicator can be learned as an additional output of the NN, based on the amount of motion noise in the input signals. For example, the confidence indicator is a percentage of chance that the calculated heart rate (HR) or breathing rate (BR) is not erroneous. For example, the confidence indicator is an incertitude calculated for the heart rate (HR) or breathing rate (BR).

### Encoder-decoder-type NN

A encoder-decoder-type NN architecture may comprise for instance a U-net NN. In general, the encoder-decoder-type NN architecture outputs a sampled signal having the same number of samples as the signals in input. The input comprises N input frames. Each frame comprises a number C of samples. Each sample comes from a segment of a different signal in an association of segments of different signals acquired at the same moment.

In general, the output frame comprises a number P of numerical values. The number P is the number of classes, or the number of output signals. Therefore, the output size is equal to NxP. The input size is equal to NxC, wherein C is the number of channels per input frame. When the outputs are classes, the NN is called a segmentation NN. When the outputs are signals, the NN can be referred to as a translation NN.

In a simple example, the number of channels C is equal to 1. For instance, the sensor is the radar unit 7 and the input signal only comprises the distance computed from one RX antenna, or from a beamformed combination of all the RX antennae. Therefore, an input frame comprises one sample: a sample of said distance.

For the sake of illustration, another example of input and outputs of a encoder-decoder-type NN in inference phase is represented on **Figure 9**. For instance, N= 500.

For example, on Figure 9, each input frame comprises C=3 samples. One of the input frames is represented: input frame 36, which comprises three samples: 33, 34 and 35. Each of the samples 33, 34 and 35 come from a different sampled signal.

The sample 33 comes from the raw RF signal 10. For instance, the signals are all sampled at a frequency 1/dt, wherein dt is the time interval of sampling which is represented on Figure 9.

In general, the samples in a same input frame are synchronized samples.

For the dog collar 1, the number of channels C corresponds to the number of different sensors embedded in the dog collar 1 multiplied by the dimension of their respective signals. In the example depicted on Figure 9, the NN is in an inference phase.

In the example of figure 9, the sensors of the dog collar 1 comprise at least:
- a sensor from the radar unit 7, and
- a sensor from the movement sensor unit 12.

Therefore, the number of sensors is equal to 2.

In the example depicted on Figure 9, the raw RF signal 10 measured and/or extracted from the radar unit 7 is a unidimensional signal,

The raw movement signal 13 measured and/or extracted from movement sensor unit 12 is a two-dimensional signal. The first dimension 131 and the second dimension 132 are depicted on Figure 9 by two different rectangles.

Therefore, in the example of the NN of Figure 1 in the inference phase, C=3 and the input of the NN comprises:
- the distance computed from the raw RF signal 10 measured from one RX antenna of the radar unit 7,
- the magnitude of the motion sensor of the movement sensor unit 12 (first dimension 131),
- the angular velocity of the motion sensor of the movement sensor unit 12 (second dimension 132).

Therefore, in the inference phase, an input frame comprises three samples:
- a sample of a segment of the distance computed from the raw RF signal 10,
- a sample of a segment of the magnitude of raw movement signal 13,
- a sample of a segment of the angular velocity of the raw movement signal 13.

The segmentation model outputs a single output frame 41.

In the example depicted on Figure 9, the number P is equal to two, and the output signals are as one can see the breathing peak probability signal 40 over the time, and the heart peak probability signal 39 over the time. As one can remark, the arrhythmia of the heart is depicted as the intervals between the high peak probability are not equal.

In a variant, one of the output signals of the NN may be a probability to belong to a class of a classification. For instance, the output signal is a probability signal of the activity of the dog, and the labelled activity is "at rest".

For the sake of illustration, the NN of the Figure 9 is depicted in its training phase with reference to **Figure 10****.**

In the training phase, the NN further receives in its input a segment of heartbeat reference signal 37 and a segment of breath peak reference signal 38 synchronized with the segments of the other input signals 10, 131 and 132. The segment of heartbeat reference signal 37 and a segment of breath peak reference signal 38 are measured from the training device 28.

During the training phase, the segment of heartbeat reference signal 37 and the segment of breath peak reference signal 38 are used as labels.

If the sample rates of the signals are different, interpolation resampling method may be used. In a best mode, one configures at least part of the various sensors in order to measure all the signals with a same sampling rate.

In a **third embodiment,** the encoder-decoder-type NN receives N input frames and translates them into N output frames of signal. The N input frames comprise at least N samples of a signal of the **input signal list.** The N output frames comprise at least a segment of an ECG signal sampled into N samples. In other words, the sampled segment of signal in the target domain is converted into a sampled segment of signal in the source domain, wherein the pulse detection is easier.

In a **fourth embodiment,** the encoder-decoder-type NN receives N input frames and output N output frames. The N input frames comprise at least N samples of a signal of the **input signal list.** The N output frames comprise at least a segment of a heart signal (HS) sampled into N samples. In other words, the sampled segment of signal in the source domain that comprise mixed information comprising: a heart signal, a breathing signal, motion and noise is translated into a sampled segment of clean heart signal, wherein the pulse detection is easier.

In such a case, the training phase comprises the providing of simulated data into the training data. One first generates a simulated heart signal (HS), a simulated breathing signal (BS), a simulated motion signal and a simulated noise, then one mixes the simulated signals all together. The raw NN is then fed with the training data and with the corresponding simulated heart signal (HS) until the NN is trained and ready for the inference phase.

In a **fifth embodiment,** the encoder-decoder-type NN receives N input frames and output N output frames. The N input frames comprise at least N samples of a signal of the **input signal list.** The N output frames comprise at least a segment of a breathing signal (BS) sampled into N samples. The fifth embodiment is similar to the fourth embodiment. The only difference is that the NN is trained to determine the breathing signal (BS) instead of the heart signal (HS).

In a **sixth embodiment,** the encoder-decoder-type NN performs denoising and source separation. The encoder-decoder-type NN receives N input frames and output N output frames. The N input frames comprise at least N samples of a signal of the input signal list.

An example source separation as in the sixth embodiment is depicted on **Figure 11****.** An input frame 43 is depicted. As one can see, the inputs of the NN comprise C signals from the input signal list.

For instance, C= 1 and the signal is the phase signal 42 calculated by beamforming.

For instance, C= 2 and the signals are the phase signal 42 calculated by beamforming and the velocity signal calculated by beamforming.

For instance, C= 6 and the signals are: the 3-dimensional phase signal 42 and the 3-dimensional acceleration.

In all cases, the phase signal 42 is noisy, as depicted on the Figure 11. Indeed, the phase signal 42 comprises the mixed information of heart signal (HS), breathing signal (BS), movements and noise. In other words, the sources of the signal are mixed and should be separated.

In an inference phase, the NN outputs N output frames. P=2 and each output frame 46 comprises a sample of a segment of breathing signal 44 (BS) and a sample of a segment of heart signal 45 (HS).

In all cases, from the heart signal (HS) and/or the breathing signal (BS), the heart rate (HR) and the breathing rate (BR) can be then calculating with standard algorithms or with an artificial intelligence software such as a supplementary NN2 or supplementary layers of the NN.

For instance, in a **seventh embodiment** represented on **Figure 12****,** the supplementary NN2 is a encoder-decoder-type NN configured to execute a peak segmentation. The encoder-decoder-type NN receives N input frames. The N input frames correspond to a sampled segment of a calculated heart signal (HS) and a sampled segment of calculated breathing signal (BS). For instance, the calculated heart signal (HS) and calculated breathing signal (BS) are calculated by applying the methods described in relation with the third, fourth, fifth or sixth embodiments.

The encoder-decoder-type NN output N output frames, wherein an output frame comprises at least a sampled probability that the corresponding sample of the calculated heart signal (HS) is a peak. For instance, the output frame further comprises a sampled probability that the corresponding sample of the calculated breathing signal (BS) is a peak.

Therefore, one can determine peaks of heart signal (HS) and breathing signal (BS) by using NN in series.

### Multi-task NN

In an **eight embodiment,** represented with reference to **Figure 13****,** the neural network unit 16 comprises a multi-task NN. A multi-task NN is a NN which mutualized some tasks into a backbone 29 and has several different heads 30, 31 for outputting different outputs from the very same inputs.

For instance, in order to make easier the regression described with reference to the first and second embodiments, i.e. in order to extract the heart rate (HR) and the breathing rate (BR), a supplementary task may be learned by the neural network unit 16.

For instance, the supplementary task is a source separation executed similarly to the sixth embodiment, in order to extract the heart signal (HS) and the breathing signal (BS).

In such a case, the multi-task NN has two heads 30, 31: one head 31 is dedicated to the extraction of the heart rate (HR) and the breathing rate (BR) by regression, and the other head 30 is dedicated to the extraction of the heart signal (HS) and the breathing signal (BS).

Such a multi-task NN allows better extraction quality and is easier to embed into the dog collar 1.

The multi-task NN receives N input frames. The N input frames comprise at least N samples of a signal of the input signal list. Namely, the signal comprises mixed information from the heart beating, the breathing, movements and noise.

In the example depicted on Figure 13, the multi-task NN outputs:
- N output frames comprising the heart signal (HS) and the breathing signal (BS), and
- the heart rate (HR) and the breathing rate (BR).

In another example, the multi-task NN outputs:
- N output frames comprising the heart signal (HS) and the breathing signal (BS), and
- N output frames comprising the heart peak probability signal and/or the breathing peak probability signal.

In the other example, the neural network unit 16 may be further configured to count the number of heart peaks and breathing peaks in order to calculate the heart rate (HR) and the breathing rate (BR).

### Other examples

In all the preceding embodiments and/or examples, by the wording "heart rate (HR)" or "breathing rate (BR)", one would understand that the rate is a **mean rate** calculated on the duration of the raw input segments.

In all the preceding embodiments and/or examples, when the NN calculates the heart signal (HS), the breathing signal (BS), the heart peak probability signal and/ or the breathing peak probability signal, one would understand that a further task may be advantageously performed, in order to extract an **instantaneous rate:** the heart rate or the breathing rate.

Indeed, the instantaneous heart rate is the inverse function of the temporal distance between two heart peaks. The instantaneous breathing rate is the inverse function of the temporal distance between two breathing peaks.

The further task of calculating the instantaneous heart rate or breathing rate may be performed by an algorithm. In another example, the further task may be also performed directly by the NN, for instance in the output layers.

The instantaneous rate is outputted as a signal rate comprising successive numerical values of the rate corresponding to the different values of the rate over time.

Thanks to the instantaneous rate, it is then possible to calculate the variability of the rate. Similarly, the variability calculation may be performed by a dedicated algorithm or preferably directly performed by the NN. The rate variability is very useful. For instance, heart rate variability (HRV) helps determining when the dog suffers arrhythmia.

In all the preceding embodiments and/or examples, it has been described a simultaneous extraction of the heart rate (HR) and the breathing rate (BR), as well as a simultaneous extraction of the heart signal (HS) and the breathing signal (BS). One will understand the very same calculations as described may be performed for extracting the heart rate (HR) only, or the heart signal (HS) only, that is to say without extracting the breathing rate (BR) or the breathing signal (BS).

Similarly, in all the preceding examples, it has been described a simultaneous extraction of the heart peak probability signal and the breathing peak probability signal. One will understand the very same calculations as described may be performed for extracting only one of the two probability signals.

Similarly, most of the preceding examples related to input signals comprising raw RF signals 10, and raw movements signals 13. On will understand that similar calculations can be performed with input signals comprising only raw RF signals 10, only raw movements signals 13, or a combination of raw RF signals 10, and/or raw movements signals 13 and/or signals from further sensing units.

### Method

In general and with reference to **Figure 14****,** a method for providing a dog collar 1 is described.

In a first step 47, a training collar and a training device 28 as described hereinabove are provided.

In a second step 48, both are worn by a dog during a data collection phase wherein the measured data are synchronously recorded into a same memory card of the training collar. Hence, the synchronization of the data incoming from the training collar and from the training device is easier. Moreover, the memory is for instance a SD card. Therefore, it is possible to record continuously during long collection phases without interruption. Then, the same data collection phase is reiterated with another dog. Same is performed for a plurality of dogs.

In a third step 49, a raw neural network is provided.

In a fourth step 50, the raw neural network is trained by the collected data in the various manners described hereinabove.

In a fifth step 51, the trained neural network is recorded in the storage module 5 of the dog collar 1 as described hereinabove.

## Claims

1. An intelligent dog collar for monitoring physiological parameters of a dog, comprising:
- a movement sensor unit (12) comprising an accelerometer and/or a gyrometer, wherein the movement sensor unit (12) is configured to detect raw movement signals (13) of the dog collar (1),
- a storage module (15) storing a trained neural network (16), the neural network (16) being configured to determine a physiologic information into raw movement signals (13) detected by the movement sensor unit (12),
- a processing unit (5) connected to the movement sensor unit and configured to operate the trained neural network,
- a memory (6) configured to store the identified physiologic information,
- an interface (17) for transmitting to a communication device (18) the identified physiologic information.

2. A dog collar according to claim 1, further comprising a transmitting antenna adapted to emit a radiofrequency (RF) detecting signal having a frequency comprised between 57 GHz and 81 GHz, and at least a receiving antenna adapted to detect a raw RF signal (10) which comprises reflection of the RF detecting signal, wherein the neural network (16) is further configured to determine the physiologic information into raw movement signals (13) thanks to the raw RF signals (10).

3. A dog collar according to one of claim 1 or claim 2, wherein the neural network (NN) performs a regression function, and wherein the physiologic information comprises a heart rate (HR) of the dog or a breathing rate (BR) of the dog.

4. A dog collar according to one of claim 1 to claim 3, wherein the neural network (NN) has an encode-decoder architecture enabling signal translation or segmentation, and wherein the physiologic information comprises a heart signal (HS) of the dog and/or a breathing signal (BS) of the dog and/or a heart peak probability signal of the dog and/or breathing peak probability signal of the dog.

5. A dog collar according to claim 4, wherein the neural network performs a source separation, and the physiologic information comprises both a heart signal (HS) of the dog and a breathing signal (BS) of the dog.

6. A dog collar according to claim 3 and claim 4 or claim 5, wherein the neural network (NN) is a multi-task neural network, the neural network (NN) comprising a backbone of shared layers and two heads of task-specific layers, one of the head comprising the regression function and the other head comprising the segmentation function, or the translation function.

7. A distributed system comprising a fleet of dog collars (1) according to one of claim 1 to claim 6, a cloud application and a data repository configured to store raw movement signals (13) and physiologic information collected from the fleet.

8. A method for providing a dog collar (1) for monitoring physiological parameters of a dog, the method comprising:
- providing (S1) a training collar comprising a movement sensor unit (12) comprising an accelerometer and/or a gyrometer, wherein the movement sensor unit (12) is configured to detect raw movement signals (13) of the dog collar (1),
- providing (S2) a training device (28) adapted to be worn by a dog on a body part (28, 281, 282) of the dog and configured to detect a heartbeat reference signal of the dog,
- training (S3) a neural network (NN), wherein the training comprises:
the acquisition (S31) of training data, comprising, for each dog of a population of dogs,
* detecting a raw movement signal segment and a heartbeat reference signal (111) segment during the simultaneous wearing, by the dog, of both the training collar (1) and the training device (28),
* determining peaks in the heartbeat reference signal (111) segment and labeling said peaks in the heartbeat reference signal segments,
* storing the raw movement signal (13) segment in association with the heartbeat reference signal (111) segment and the labeled peaks,
feeding (S32) the neural network with said training data, in order to train the neural network for a task, wherein the task comprises an determination of a physiologic information from raw movement signals detected by the movement sensor unit,
recording (S4), in a storage module (15), the trained neural network (NN), and providing a dog collar (1) comprising a movement sensor unit (12) as the movement sensor unit of the training collar, and the storage module comprising the trained neural network (NN).

9. The method according to claim 8, wherein the training further comprises : - determining an activity state in the raw movement signal segment and labeling said activity state in the raw movement signal segment, the activity state comprising at least a rest state of the dog,
- storing the raw movement signal segment in association with the labeled activity state, the training data further comprising said raw movement signal segment and labeled activity state,
the method further comprising training the neural network to determine a rest state of the dog into raw movement signals detected by the movement sensor unit (12).

10. The method according to claim 8 or claim 9, wherein the body part of the dog is the ear, the groin or the chest.

11. The method according to one of claim 8 to claim 10, wherein the training device is an electrocardiogram (ECG) and the heartbeat reference signal is a ECG.

12. The method according to one of the claims 8 to 11, wherein dog collar (1) further comprises a transmitting antenna adapted to emit a radiofrequency (RF) detecting signal having a frequency comprised between 57 GHz and 81 GHz, and at least a receiving antenna adapted to detect a raw RF signal (10) which comprises reflection of the RF detecting signal,
wherein the training further comprises:
- detecting a raw RF signal segment during said simultaneously wearing;
- storing the RF signal segment in association with the raw movement signal the heartbeat reference signal (111) segment and the labeled peaks.

13. The method according to claim 12, wherein the raw RF signal is pre-processed to extract the displacement (d) and/or the velocity of the skin and/or of the dog hairs of the throat of the dog.

14. The method according to one of the claims 8 to 13, wherein the trained task comprises a regression function, and wherein the physiologic information comprises the heart rate or the breathing rate of the dog.

15. A method according to one of the claims 8 to 14, wherein the neural network has an encoder-decoder architecture, and is trained to output the physiologic information, wherein the physiologic information comprises the heart signal (HS) or the breathing signal (BS) or a heart peak probability signal or a breathing peak probability signal.
